# EUROPÄISCHE PATENTSCHRIFT

(11) **EP 1 411 845 B1**
(45) Veröffentlichungstag und Bekanntmachung des Hinweises auf die Patenterteilung: **11.09.2013**
(21) Anmeldenummer: 02767280.7
(22) Anmeldetag: 31.07.2002
(51) Int. Cl.: A61B 17/32, A61B 17/28, B25B 7/06

(54) **MEDIZINISCHES INSTRUMENT MIT ZWEI TEILEN MIT VERBINDUNGSVORRICHTUNG ZU DEREN VERBINDUNG**
MEDICAL INSTRUMENT CONSISTING OF TWO PARTS WITH A CONNECTING DEVICE FOR THE CONNECTION THEREOF
INSTRUMENT MEDICAL COMPORTANT DEUX PARTIES AINSI QU'UN DISPOSITIF DE LIAISON DESTINE A LES RELIER

(30) Priorität: 04.08.2001 DE 10138394
(43) Veröffentlichungstag der Anmeldung: 28.04.2004
(73) Patentinhaber: Aesculap AG, 78532 Tuttlingen (DE)
(72) Erfinder: DWORSCHAK, Manfred, 78589 Dürbheim (DE); LUTZE, Theodor, 78582 Balgheim (DE); MORALES, Pedro, 78532 Tuttlingen-Nendingen (DE); WEISSHAUPT, Dieter, 78194 Immendingen (DE)
(74) Vertreter: Regelmann, Thomas
(86) Internationale Anmeldenummer: PCT/EP2002/008495
(87) Internationale Veröffentlichungsnummer: WO 2003/013377

(56) Entgegenhaltungen:
- DE-U- 20 006 450
- DE-U- 29 708 218
- FR-A- 760 671
- US-A- 3 735 763
- US-A- 5 316 512

## Beschreibung

Die Erfindung betrifft ein medizinisches Instrument mit einem ersten Teil und einem zweiten Teil, welche über eine Verbindungsvorrichtung miteinander verbunden sind, wobei das zweite Teil an dem ersten Teil anliegt, und mit einem Deckelelement, welches mit dem ersten Teil verbunden ist und welches auf das zweite Teil so aufgelegt ist, daß die Relativbewegung des zweiten Teils von dem ersten Teil weg über das Deckelelement gesperrt ist, wobei ein Teil mit einem Zapfen versehen ist und das andere Teil mit einer Zapfenausnehmung zur Aufnahme des Zapfens versehen ist.

Es kann sich dabei beispielsweise um eine Zange insbesondere in der Form eine Klemme handeln.

Aus der DE 200 06 450 U1 ist ein ineinandertreffendes Handwerkzeug aus einem männlichen und weiblichen Griff bekannt, wobei der weibliche Griff an der Verbindungsstelle eine Nut aufweist, deren Wände jeweils mit einem Achsloch versehen sind, an die ein kegelförmiger Stempel gedrückt wird, wodurch eine ausgeweitete Nut entsteht.

Aus der US 3,735,763 ist ein Hemostat bekannt, welcher ein erstes Griffteil und ein zweites Griffteil umfaßt. Jeder Griffteil ist mit einem Schwenkverbindungsbereich versehen.

Aus der US 5,316,512 ist ein Lampenzieher bekannt.

Aus der FR 716 671 ist eine Zange bekannt.

Aus der DE 297 08 218 U1 ist eine chirurgische Klemme mit zwei gegeneinander schwenkbaren, sich überkreuzenden Armen bekannt, die in ihrem Überkreuzungsbereich dadurch relativ zueinander geführt sind, dass ein Arm eine längliche Öffnung im anderen Arm durchgreift. Die längliche Öffnung ist an der Längsseite von einem brückenförmigen Steg überdeckt, der durch einen bis in die längliche Öffnung reichenden Einschnitt unterbrochen ist.

Der Erfindung liegt die Aufgabe zugrunde, ein medizinisches Instrument der eingangs genannten Art zu schaffen, bei welchem die Verbindung auf einfache und kostengünstige Weise herstellbar ist.

Diese Aufgabe wird erfindungsgemäß dadurch gelöst, dass der Zapfen an dem entsprechenden Teil einstückig angeformt ist, dass der Zapfen an dem entsprechenden Teil einstückig angeformt ist, dass die Zapfenausnehmung einen verbreiterten Bereich aufweist, der einen größeren Querschnitt als der Zapfen hat und in dem das Deckelelement angeordnet ist, dass das Deckelelement so ausgebildet ist, dass es nicht über eine Oberseite des zweiten Teils hinausragt, und dass an der Zapfenausnehmung durch den verbreiterten Bereich eine Anlagefläche für das Deckelelement an das zweite Teil angeordnet ist.

Die Anlage des zweiten Teils an dem ersten Teil sperrt die Bewegung des zweiten Teils zum ersten Teil hin und das Deckelelement sperrt die Bewegung in die Gegenrichtung. Dadurch lässt sich eine sichere und insbesondere im Wesentlichen spielfreie Verbindung zwischen den beiden Teilen ermöglichen. Andererseits läßt sich dann aber weiterhin noch eine Relativbewegung der beiden Teile in Querrichtungen zu dieser Verbindungsrichtung einstellen, indem insbesondere entsprechende Führungen vorgesehen werden. Beispielsweise läßt sich so eine Linearverscheiblichkeit zwischen den beiden Teilen herstellen oder die beiden Teile lassen sich relativ zueinander drehbar ausbilden.

Die entsprechende Verbindungsvorrichtung läßt sich auf einfache Weise auch bei einem medizinischen Instrument, welches im wesentlichen aus einem Kunststoffmaterial hergestellt ist, einsetzen. Beispielsweise läßt sich das Deckelelement mit dem ersten Teil über Verklebung, Schweißen, einen Schnappverschluß oder dergleichen verbinden.

Ein entsprechendes Instrument läßt sich auch auf einfache Weise herstellen, da zuerst die beiden Teile aneinander angelegt werden und das Deckelelement dann diese Stellung sichert. Das Deckelelement läßt sich nach dem Aneinanderlegen der beiden Teile auflegen.

Ein Teil ist mit einem Zapfen versehen und das andere Teil mit einer Zapfenausnehmung zur Aufnahme des Zapfens. Auf diese Weise läßt sich insbesondere eine Führung für das zweite Teil relativ zu dem ersten Teil über die Verbindungsvorrichtung ausbilden.

Insbesondere ist es vorteilhaft, wenn ein Oberflächenbereich des zweiten Teils an einem zugeordneten Oberflächenbereich des ersten Teils anliegt, um so die Relativbewegung des zweiten Teils zu dem ersten Teil hin zu sperren.

Weiterhin ist es vorteilhaft, wenn ein Oberflächenbereich des Deckelelementes an einem zugeordneten Oberflächenbereich des zweiten Teils anliegt, um so eine Relativbewegung des zweiten Teils vom ersten Teil weg zu sperren.

Bei einem ersten Ausführungsbeispiel ist das Deckelelement auf dem Zapfen aufgesetzt. Der Zapfen dient dann einerseits zur Führung der beiden Teile relativ zueinander, beispielsweise zur Ausbildung einer Schwenkführung, und andererseits läßt sich das Deckelelement dann mit dem Zapfen verbinden, um die Verbindung zwischen den beiden Teilen zu fixieren.

Grundsätzlich läßt sich das Deckelelement auf verschiedenartige Weisen mit dem ersten Teil und insbesondere mit dessen Zapfen verbinden, beispielsweise über Klebung, Verschraubung, Schweißen oder thermische Verbindungsverfahren. Ein erfindungsgemäßes medizinisches Instrument läßt sich auf einfache Weise zusammensetzen, wenn das Deckelelement in der Art eines Schnappverschlusses auf den Zapfen aufsetzbar ist. Bei der Herstellung des medizinischen Instrumentes wird dann das zweite Teil mit einer Zapfenausnehmung ausgerichtet auf den Zapfen des ersten Teils an dem ersten Teil angelegt. Durch anschließendes Aufsetzen des Deckelelementes wird diese Stellung gesichert, so daß die Bewegung des zweiten Teils von dem ersten Teil in Längsrichtung des Zapfens weg gesperrt ist. Insbesondere läßt sich dann eine solche Verbindung im wesentlichen spielfrei ausbilden.

Es kann vorgesehen sein, daß der Zapfen mit einem oder mehreren Längsschlitzen versehen ist, um eine Aufnahme für das Deckelelement in der Art eines Schnappverschlusses zu bilden. Dadurch läßt es sich erreichen, daß entsprechende Teile des Zapfens beim Aufsetzen des Deckels unter Kraftbeaufschlagung zurückfedern und dann in eine entsprechende Ausnehmung des Deckels einrasten können. Der Deckel selber kann dann nur unter Kraftbeaufschlagung wieder von dem Zapfen gelöst werden.

Günstigerweise weist dabei das Deckelelement mindestens im Bereich einer Anlagefläche an das zweite Teil eine größere Querabmessung auf als der Zapfen. Dadurch ist eine entsprechende Anlagefläche bereitgestellt, über die die Wegbewegung des zweiten Teils vom ersten Teil sperrbar ist.

Korrespondierend mit der Anlagefläche des Deckelelementes ist dabei an der Zapfenausnehmung eine Anlagefläche für das Deckelelement an das zweite Teil angeordnet, um entsprechend die nötige Anlagefläche zur Sperrung der Relativbewegung bereitzustellen.

Bei einer Variante der Ausführungsform ist der Zapfen an dem ersten Teil angeordnet und die Zapfenausnehmung an dem zweiten Teil angeordnet. Aus diese Weise läßt sich eine relative Drehbarkeit zwischen den beiden Teilen realisieren.

Bei einem zweiten Ausführungsbeispiel weist das erste Teil eine Aufnahme auf, in welcher das zweite Teil mindestens teilweise an dem ersten Teil anlegbar ist.

Das Deckelelement sperrt dann ein Austreten des zweiten Teils aus der Aufnahme, das heißt das zweite Teil liegt an einer zugewandten Fläche des Deckelelementes an.

Es kann vorgesehen sein, daß das zweite Teil in einer Aufnahme relativ zum ersten Teil beweglich ist. Die Aufnahme kann dabei in dem zweiten Teil selber gebildet sein oder in dem ersten Teil. Insbesondere ist dabei eine Führung des zweiten Teils an dem ersten Teil vorgesehen, wobei es sich beispielsweise um eine Linearführung oder um eine Schwenkführung handeln kann.

Es kann vorgesehen sein, daß ein Teil mindestens ein Zapfen und das andere Teil mindestens eine Aufnahme zum Führen oder Halten des anderen Teils an dem Teil aufweist. Ist beispielsweise ein Zapfen drehbar in einer entsprechenden Zapfenaufnahme gehalten, so ist damit eine Schwenkführung ausgebildet. Ist ein Zapfen in einer entsprechenden Aufnahme, welche in der Art eines Längsschlitzes ausgebildet ist, gelagert, dann läßt sich damit eine Verschiebungsführung ausbilden. Sind beabstandete Zapfen in entsprechende Ausnehmungen des anderen Teils eingetaucht (oder weist ein Teil einen Zapfen und eine Ausnehmung auf und das andere Teil eine korrespondierende Ausnehmung und einen korrespondierenden Zapfen), dann ist dadurch bei hergestellten Zapfen-Zapfenausnehmung-Verbindungen die Beweglichkeit in der Aufnahme gesperrt und die beiden Teile sind unbeweglich aneinander gehalten.

Insbesondere sind die beiden Teile über die Verbindungsvorrichtung relativ zueinander beweglich angeordnet. Beispielsweise ist entsprechend über einen Zapfen als Welle eine Schwenkführung ausgebildet.

Die beiden Teile sind dann drehbar miteinander verbunden. Auf diese Weise läßt sich mittels der erfindungsgemäßen Verbindungsvorrichtung eine Zange und als Sonderform dann beispielsweise eine Klemme ausbilden.

Durch eine feste Verbindung zwischen einem ersten Teil und einem zweiten Teil läßt sich aber auch eine Pinzette ausbilden.

Ganz besonders vorteilhaft ist es, wenn das erste Teil und/oder das zweite Teil aus einem Kunststoffmaterial hergestellt ist und jeweils insbesondere einstückig ausgebildet sind. Die entsprechende Verbindungsvorrichtung läßt sich dann auf fertigungstechnisch einfache Weise herstellen.

Es kann grundsätzlich vorgesehen sein, daß das Deckelelement ein getrenntes Teil ist, welches mit dem ersten Teil beispielsweise über Verklebung, Verschweißen oder über eine Rastverbindung verbindbar ist. Es kann aber auch vorgesehen sein, daß das Deckelelement einstückig an dem ersten Teil sitzt und dabei insbesondere bei der Herstellung des ersten Teils integral mithergestellt wird. Dadurch kann ein entsprechendes medizinisches Instrument allein aus zwei getrennten Teilen, nämlich dem ersten Teil und dem zweiten Teil, hergestellt werden, ohne daß darüber hinaus noch zusätzliche Teile notwendig sind.

Insbesondere sitzt dann das Deckelelement zur Auflegbarkeit auf das zweite Teil schwenkbar an dem ersten Teil. Es läßt sich dann das Deckelelement so weit beispielsweise von einer Aufnahme für das zweite Teil weg bewegen, daß dieses eben in die Aufnahme oder dergleichen einlegbar ist. Wird dann das Deckelelement auf das zweite Teil zu geschwenkt, dann läßt sich dieses auf das zweite Teil auflegen, um so wiederum die Wegbewegung des zweiten Teils zu sperren.

Auf einfache Weise läßt sich das Deckelelement schwenkbar an dem ersten Teil ausbilden, wenn es über ein Filmscharnier an dem ersten Teil sitzt.

Das einstückig an dem ersten Teil angeordnete Deckelelement läßt sich, um eine Sperrverbindung für das zweite Teil herzustellen, an dem ersten Teil fixieren, d. h. insbesondere über einen Verbindungsbereich des Deckelelements an dem ersten Teil fixieren, mit welchem das Deckelelement nicht einstückig mit dem ersten Teil verbunden ist. Das Deckelelement ist dann so an dem ersten Teil fixierbar, daß eine Wegbewegung von diesem gesperrt ist, um so wiederum durch Auflage auf das zweite Teil eine Wegbewegung des zweiten Teils zu sperren. Diese Fixierung über den Verbindungsbereich verbindet dabei das freie Ende des Deckelelements, wenn dieses schwenkbar an dem ersten Teil sitzt, mit dem ersten Teil.

Auf fertigungstechnisch und herstellungstechnisch einfache Weise läßt sich das Deckelelement an dem ersten Teil fixieren, wenn eine Rastverbindung vorgesehen ist. Diese kann auf schnelle Weise hergestellt werden, ohne daß eine aufwendige Bearbeitung wie beispielsweise beim Verschweißen notwendig ist oder daß Adhäsionsmittel vorgesehen werden müssen.

Es ist auch günstig, wenn das Deckelelement ein Kopplungselement und das erste Teil ein korrespondierendes Kopplungselement aufweisen, welche so aufeinander angepaßt und abgestimmt sind, daß bei fixiertem Deckelelement eine Querbewegung des Deckelelements relativ zum ersten Teil bezogen auf eine Aufsetzrichtung durch Kooperation der Kopplungselemente gesperrt ist. Durch diese korrespondierenden Kopplungselemente lassen sich Querkräfte auf das erste Teil quer zur Aufsetzrichtung aufnehmen. Dadurch muß die Rastverbindung die Querkräfte zumindest nicht vollständig aufnehmen und bei entsprechender Ausbildung der Kopplungselemente läßt sich der überwiegende Teil der Querkräfte durch diese abfangen. Dadurch läßt sich die Rastverbindung bezüglich ihrer Funktion als Fixierung zwischen Deckelelement und erstem Teil optimieren. Dadurch wiederum läßt sich der Zusammenbau eines erfindungsgemäßen medizinischen Instruments erleichtern, da die Fixierungsverbindung zwischen den beiden Teilen auf einfache Weise herstellbar ist.

Bei einer fertigungstechnisch günstigen Ausführungsform kooperieren die Kopplungselemente als Aufnahme und korrespondierendes Eintauchelement in die Aufnahme, um so Querkräfte aufnehmen zu können und damit in erheblichem Maße von der Rastverbindung abfangen zu können. Ein solches erstes Teil mit entsprechendem Deckelelement läßt sich auch auf einfache Weise einstückig herstellen.

Die nachfolgende Beschreibung bevorzugter Ausführungsformen dient im Zusammenhang mit der Zeichnung der näheren Erläuterung der Erfindung. Es zeigen:
- Figur 1: eine Draufsicht auf eine Klemme, welche mit einer erfindungsgemäßen Verbindungsvorrichtung versehen ist;
- Figur 2: eine Schnittansicht durch die Verbindungsvorrichtung gemäß Figur 1;
- Figur 3: eine Teilansicht eines medizinischen Instruments, welches mit einem zweiten Ausführungsbeispiel einer Verbindungsvorrichtung, welche nicht unter die Ansprüche fällt, versehen ist;
- Figur 4: eine Schnittansicht längs der Linie A-A gemäß Figur 3;
- Figur 5: eine Schnittansicht längs der Linie B-B gemäß Figur 3 in einer Teildarstellung in der Art einer Explosionsansicht;
- Figur 6: eine Teilansicht eines endoskopischen Instruments, bei dem ein erstes Teil und ein zweites Teil mit einer erfindungsgemäßen Verbindungsvorrichtung verbunden sind;
- Figur 7: eine seitliche Teilansicht eines medizinischen Instruments, welches mit einem dritten Ausführungsbeispiel einer Verbindungsvorrichtung, welche nicht unter die Ansprüche fällt, versehen ist, bei nicht fixiertem Deckelelement;
- Figur 8: die gleiche Ansicht wie Figur 7 bei fixiertem Deckelelement;
- Figur 9: eine Schnittansicht längs der Linie 9-9 gemäß Figur 8 und
- Figur 10: eine perspektivische Teilansicht der Verbindungsvorrichtung gemäß dem dritten Ausführungsbeispiel.

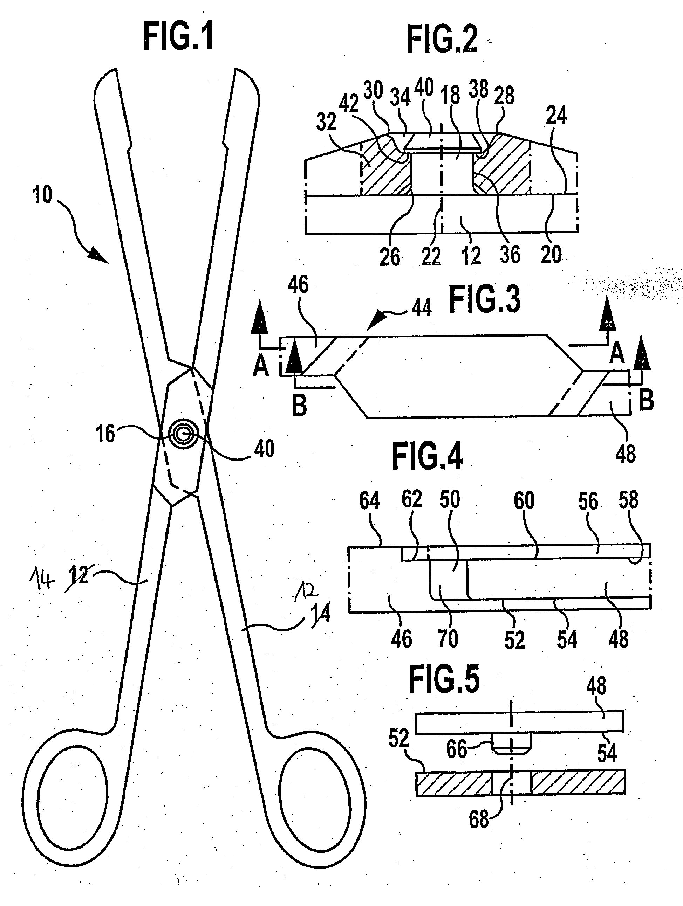

Ein Ausführungsbeispiel eines medizinischen Instruments ist durch eine Klemme gebildet, welche in Figur 1 als Ganzes mit 10 bezeichnet ist. Diese umfaßt als erstes Teil 12 einen ersten Schenkel und als zweites Teil 14 einen zweiten Schenkel. Diese sind über eine Verbindungsvorrichtung 16 miteinander verbunden, wobei diese Verbindungsvorrichtung 16 ein Gelenk umfaßt, so daß das erste Teil 12 und das zweite Teil 14 relativ zueinander verschwenkbar sind.

An dem ersten Teil 12 ist dabei ein Zapfen 18 angeordnet, welcher über eine dem zweiten Teil 14 zugewandte Oberseite 20 des ersten Teils 12 hinaussteht (Figur 2). Dieser Zapfen 18 ist einstückig an dem ersten Teil 12 angeformt; beispielsweise ist das erste Teil 12 (und auch das zweite Teil 14) aus einem Kunststoffmaterial gefertigt und dabei insbesondere jedes Teil 12, 14 einstückig ausgebildet und während der Herstellung des ersten Teils 12 beispielsweise über ein Spritzgußverfahren wird durch entsprechende Gestaltung der Gießform der Zapfen 18 mit ausgebildet.

Er kann aber auch nachträglich an dem ersten Teil 12 montiert werden, beispielsweise indem der Zapfen 18 mit einem Gewinde versehen ist und in das erste Teil 12 eingeschraubt wird. Dies ist jedoch nicht Teil der Erfindung.

Der Zapfen 18 erstreckt sich dabei in einer Längsrichtung 22 auf der Oberseite 20 des ersten Teils 12.

Das zweite Teil 14 weist eine Unterseite 24 auf, welche an die Oberseite 20 des ersten Teils 12 angepaßt ist, so daß das zweite Teil 14 mit seiner Unterseite 24 an der Oberseite 20 des ersten Teils 12 anliegen kann. Durch eine solche Anlage ist die Relativbewegung des zweiten Teils 14 zu dem ersten Teil 12 hin gesperrt.

Der Zapfen 18 kann herstellungsbedingt am Übergang zu der Oberseite 20 des ersten Teils 12 eine verbreiterte Flanke 26 aufweisen, so daß ein Querschnitt des Zapfens 18 im Bereich der Flanke 26 größer ist als außerhalb dieser Flanke 26.

Insbesondere ist der Zapfen 18 rotationssymmetrisch um die Achse 22 ausgebildet.

Das zweite Teil 14 weist eine Zapfenausnehmung 28 zur Aufnahme des Zapfens 18 auf. Diese erstreckt sich insbesondere zwischen der Unterseite 24 des zweiten Teils 14 und einer Oberseite 30 von diesem. In einem Bereich 32 des zweiten Teils 14 um die Zapfenausnehmung 28 weist dabei das zweite Teil 14 eine größere Dicke auf als außerhalb des Bereiches 32.

Die Zapfenausnehmung 28 ist angepaßt an die Abmessungen des Zapfens 18, so daß dieser von der Zapfenausnehmung 28 aufgenommen werden kann. Insbesondere ist die Zapfenausnehmung 28 zu der Unterseite 24 zu verbreitert, so daß auch die Flanken 26 von der Zapfenausnehmung 28 aufgenommen werden können.

Die Zapfenausnehmung 28 weist an der Oberseite 20 einen verbreiterten Bereich 34 auf, der einen größeren Querschnitt hat als der Zapfen 18 und derjenige Bereich 36 der Zapfenausnehmung 28, in dem der Zapfen 18 mit Wänden im wesentlichen parallel zur Richtung 22 geführt ist. Dadurch ist an der Zapfenausnehmung 28 eine Anlagefläche 38 gebildet, die insbesondere ringförmig ausgestaltet ist.

Auf den Zapfen 18 ist ein Deckelelement 40 aufgesetzt, welches mit einer der Oberseite 20 des ersten Teils 12 zugewandten Oberfläche 42 an der Anlagefläche 38 anliegt. Der entsprechende Teil der Oberfläche 42, welcher eine Anlagefläche des Deckelelementes 40 bildet, ist dabei bei einer ringförmigen Anlagefläche 38 ebenfalls ringförmig ausgebildet.

Das Deckelelement 40 ist insbesondere so ausgebildet, daß es in dem Bereich 34 der Zapfenausnehmung 28 angeordnet ist, so daß es nicht über die Oberseite 30 des zweiten Teils 14 hinaus ragt.

Das Deckelelement 40 kann mit dem Zapfen 18 verklebt sein, mit diesem verschweißt sein, mit diesem verschraubt sein oder durch eine thermische Bindung mit diesem verbunden sein.

Bei einer Variante einer Ausführungsform ist das Deckelelement 40 auf den Zapfen 18 in der Art eines Schnappverschlusses aufgeclipst. Es kann dann vorgesehen sein, daß der Zapfen 18 mit entsprechenden Schlitzen versehen ist, so daß das Deckelelement 40 in eine entsprechende Ausnehmung des Zapfens 18 einführbar ist und dann dort durch Einschnappen in der Art eines Schnappverschlusses gehalten ist.

Die Verbindung des zweiten Teils 14 mit dem ersten Teil 12 wird über die Verbindungsvorrichtung 16 so hergestellt, daß das zweite Teil 14 mit seiner Zapfenausnehmung 28 ausgerichtet auf den Zapfen 18 auf das erste Teil 12 gelegt wird. Dadurch ist die Bewegung des zweiten Teils 14 in Richtung des ersten Teils 12 gesperrt. Anschließend wird das Deckelelement 40 auf den Zapfen 18 aufgesetzt und dort fixiert, beispielsweise mittels eines Schnappverschlusses. Dadurch wiederum drückt die Anlagefläche der Oberfläche 42 des Deckelelementes 40 auf die Anlagefläche 38 im Bereich der Zapfenausnehmung 28 des zweiten Teils 14. Dadurch ist die Wegbewegung des zweiten Teils 14 von dem ersten Teil 12 gesperrt und es ist eine sichere Fixierung des zweiten Teils 14 an dem ersten Teil 12 erreicht.

Bei dem in den Figuren 1 und 2 gezeigten Ausführungsbeispiel ist die Verbindungsvorrichtung 16 als Gelenk ausgebildet, das heißt, das zweite Teil 14 läßt sich über den Zapfen 18 in der Zapfenausnehmung 28 als Drehlager für die Drehwelle 18 relativ zu dem ersten Teil 12 verschwenken.

Bei einem zweiten Ausführungsbeispiel einer Verbindungsvorrichtung, welche nicht unter die Ansprüche fällt und in Figur 3 als Ganzes mit 44 bezeichnet ist, sind ein erstes Teil 46 und ein zweites Teil 48 miteinander verbunden. In dem ersten Teil 46 ist dabei eine Aufnahme 50 für das zweite Teil gebildet (Figur 4). Diese Aufnahme 50 weist eine Anlagefläche 52 für das zweite Teil 48 auf, so daß eine Unterseite 54 des zweiten Teils 48 an der Anlagefläche 52 anlegbar ist. Dadurch wird die Bewegung des zweiten Teils 48 in Richtung des ersten Teils 46 gesperrt. An dem ersten Teil 46 sitzt ein Deckelelement 56, welches mit einer Oberfläche 58 an einer Oberseite 60 des zweiten Teils 48 anliegt. Dadurch ist die Bewegung des zweiten Teils 48 von dem ersten Teil 46 weg gesperrt und die Verbindungsvorrichtung sorgt für eine Verbindung der beiden Teile 46 und 48.

Das Deckelelement 56 selber ist an dem ersten Teil 46 fixiert, beispielsweise über Klebung, einen Schnappverschluß, Schweißen oder eine formschlüssige Verbindung wie eine Schraubverbindung oder durch thermische Fixierung.

Bei dem in Figur 4 gezeigten Ausführungsbeispiel weist das erste Teil 46 eine Ausnehmung 62 auf, deren Höhe der Höhe des Deckelelementes 56 entspricht. In diese Ausnehmung und ein entsprechendes Gegenstück (in der Zeichnung nicht gezeigt) läßt sich dann das Deckelelement 56 einlegen und dadurch gehalten an dem ersten Teil 46 auf das zweite Teil 48 auflegen, so daß eine Oberfläche 64 des ersten Teils 12 mit dem daran fixierten Deckelelement 56 im wesentlichen keine Vorsprünge insbesondere am Übergang zu dem Deckelelement 56 aufweist.

Die Aufnahme 50 kann so ausgebildet sei, daß das zweite Teil 48 auch in Querrichtungen (in der Figur 4 senkrecht zur Zeichenebene) an Begrenzungswänden der Aufnahme 50 anliegt. Dadurch ist das zweite Teil 48 fest an dem ersten Teil 46 gehalten.

Es kann aber insbesondere, wie beispielhaft in Figur 5 gezeigt, vorgesehen sein, daß das zweite Teil 48 in der Aufnahme 50 beweglich relativ zu dem ersten Teil 46 angeordnet ist. Beispielsweise kann eine Verschiebeführung vorgesehen sein, in welcher das zweite Teil 48 beispielsweise parallel zu einer Längsrichtung des ersten Teils 46 relativ zu diesem verschieblich geführt ist (in der Zeichnung nicht gezeigt).

Insbesondere ist es vorgesehen, wie in Figur 5 gezeigt, daß das zweite Teil 48 relativ zum ersten Teil 46 drehbar ist. Dazu ist das zweite Teil 48 dem ersten Teil 46 zugewandt mit einem Zapfen 66 versehen, welcher in eine entsprechende Ausnehmung 68 des ersten Teils 46 eintaucht. Dieser Zapfen 66 stellt dann eine Drehwelle für die Schwenkführung in der Ausnehmung 68 dar. Zur Durchführung der Drehbewegung muß in der Aufnahme 50 ein entsprechender Raum 70 bereitgestellt werden (vergleiche Figur 4), so daß das zweite Teil 48 insbesondere mit einem Endbereich nicht an Begrenzungswände der Aufnahme 50 anstößt, bevor der eingestellte maximale Schwenkwinkel erreicht ist.

Ein Zapfen als Drehwelle kann alternativ an dem ersten Teil 46 in Richtung des zweiten Teils 48 angeordnet sein, wobei dann das zweite Teil 48 eine entsprechende Ausnehmung ausweist.

Ferner kann eine Mehrzahl von Zapfen und Ausnehmungen vorgesehen sein, auch alternierend, das heißt das ersten Teil 46 ist mit Ausnehmungen und Zapfen versehen und entsprechend das zweite Teils 48 mit korrespondierenden Zapfen und Ausnehmungen. Auf diese Weise läßt sich durch die Zapfen und Ausnehmungen, wenn eine Mehrzahl vorgesehen ist, das zweite Teil 48 fest an dem ersten Teil 46 halten, wobei die Wegbewegung des zweiten Teils 48 vom ersten Teil 46 durch das Deckelelement 56 gesichert ist.

Die Verbindungsvorrichtung 44 läßt sich auf einfache Weise an einem medizinischen Instrument herstellen, bei dem das erste Teil 46 und das zweite Teil 48 aus einem Kunststoffmaterial gefertigt sind.

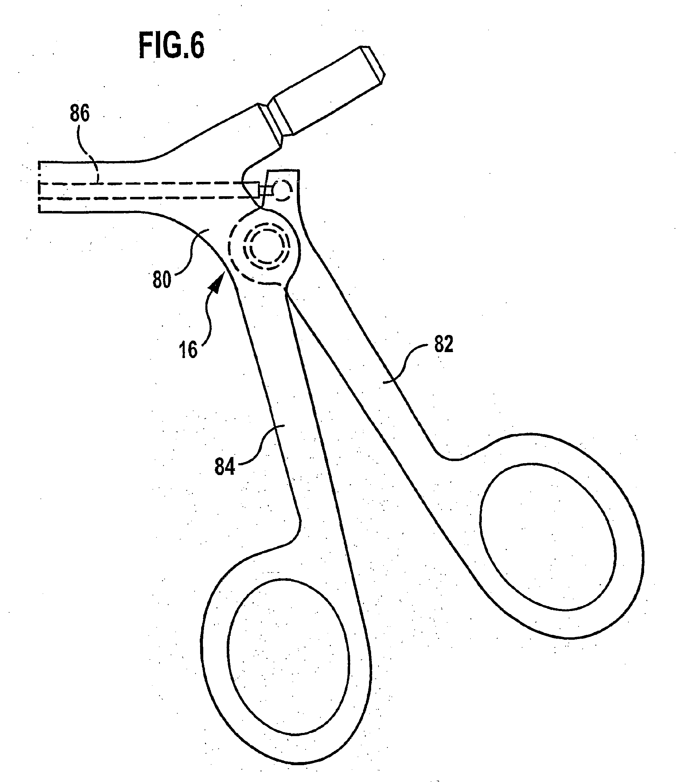

Ein weiteres Ausführungsbeispiel eines medizinischen Instruments, bei welchem ein erstes Teil 80 und ein zweites Teil 82 über eine Verbindungsvorrichtung 16 bzw. 44 miteinander verbunden sind, ist in Figur 6 gezeigt. Es handelt sich dabei um ein endoskopisches Instrument, wobei an dem ersten Teil 80 ein Griffelement 84 angeordnet ist und das zweite Teil 82 ein weiteres Griffelement darstellt, welches über die Verbindungsvorrichtung 16 bzw. 44 relativ zu dem Griffelement 84 schwenkbar ist. Über die Schwenkbewegung des Griffelements 82 läßt sich dabei ein Betätigungselement 86 in dessen Längsrichtung verschieben.

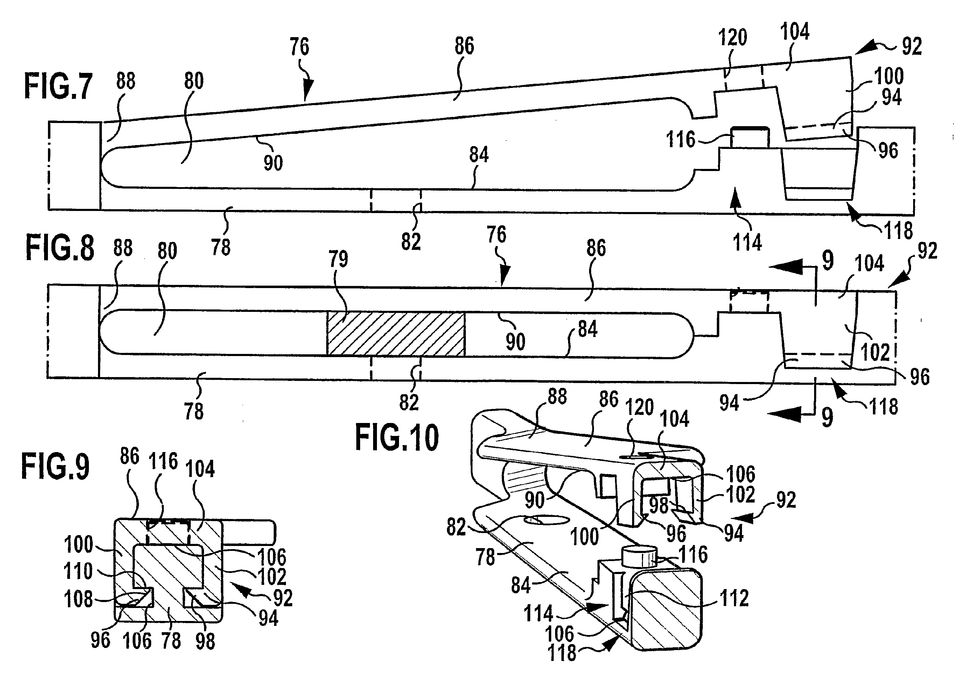

Bei einem dritten Ausführungsbeispiel einer Verbindungsvorrichtung, welche nicht unter die Ansprüche fällt und in den Figuren 7 bis 10 gezeigt ist und in Figur 7 und 8 als Ganzes mit 76 bezeichnet ist, ist an einem ersten Teil 78 eine Aufnahme 80 für ein in Figur 8 gezeigtes zweites Teil 79 gebildet. Innerhalb dieser Aufnahme 80 ist das zweite Teil 79 beispielsweise längsverschieblich oder schwenkbar angeordnet und eine entsprechende Verschiebungsführung oder Schwenkführung vorgesehen. Bei dem gezeigten Ausführungsbeispiel umfaßt das erste Teil 78 der Aufnahme 80 zugewandt eine Zapfenaufnahme 82 als Schwenklager zur Aufnahme eines an dem zweiten Teil 79 gebildeten Zapfens als Wellenstummel.

Die Aufnahme 80 ist zur einen Seite hin durch eine im wesentlichen ebene Fläche 84 begrenzt, an die das zweite Teil 79 anlegbar ist, um so eine Sperrfläche für die Relativbewegung zwischen dem zweiten Teil 79 und dem ersten Teil 78 in Richtung der Fläche 84 bereitzustellen.

An dem ersten Teil 78 sitzt einstückig mit diesem verbunden ein Deckelelement 86, welches integral bei der Herstellung des ersten Teils 78 bereitgestellt wurde. Beispielsweise ist dieses Deckelelement 86 über ein Filmscharnier 88 schwenkbar an dem ersten Teil 78 angeordnet.

Das Deckelelement 86 weist eine der Fläche 84 zugewandte, die Aufnahme 80 begrenzende, im wesentlichen ebene Fläche 90 auf, welche als Sperrfläche für die Wegbewegung des zweiten Teils 79 vom ersten Teil 78 in Richtung der Fläche 90 dient. Über die Fläche 90 oder zumindest einen Teilbereich dieser Fläche ist das Deckelelement 86 auf das zweite Teil 79 aufgelegt (Figur 8).

Das Deckelelement 86 ist an dem ersten Teil 78 über eine Rastverbindung 92 fixierbar. Dazu ist das Deckelelement 86 mit einer Rastnasenvorrichtung 94 versehen, welche an einem Ende des Deckelelements 86 angeordnet ist, die dem Verbindungsbereich des Deckelelements 86 mit dem ersten Teil 78 zu dessen einstückiger Verbindung, beispielsweise dem Filmscharnier 88, abgewandt ist.

Bei dem gezeigten Ausführungsbeispiel umfaßt die Rastnasenvorrichtung 94 seitlich gegenüberliegende Rastnasen 96, 98, welche an seitlich gegenüberliegenden beabstandeten Wänden 100, 102 gebildet sind. Diese Wände 100, 102 ragen dabei über die Fläche 90 in Richtung der Fläche 84 hinaus. Die Wände 100, 102 sind über ein Verbindungsteil 104 des Deckelelements 86 verbunden, welches eine Anlagefläche 106 zwischen den beiden Wänden 100 und 102 bereitstellt.

Die Rastnasen 96, 98 ragen aufeinander zuweisend über Innenflächen ihrer jeweiligen Wände 100, 102 hinaus.

In dem ersten Teil 78 ist für die Rastnasenvorrichtung 94 eine entsprechend ausgebildete Ausnehmung 106 gebildet, um die Rastnasen 96, 98 aufzunehmen, und zwar in der Art einer Hinterschneidung derart, daß bei in die entsprechenden Ausnehmungen eingetauchten Rastnasen eine Bewegung des Deckelelements 86 in Gegenrichtung zu einer Aufsetzrichtung (beispielsweise in Gegenrichtung zu einer Schwenkrichtung des Deckelements 86 auf die Fläche 84 zu) gesperrt ist. Insbesondere sind dazu die Rastnasen 96, 98 jeweils mit einer Schrägfläche 108 in einem Winkel zu der Fläche 90 und mit einer Parallelfläche 110 zu der Fläche 90 ausgebildet, wobei die Schrägfläche 108 mit der Parallelfläche 110 in einem spitzen Winkel liegt. Dadurch läßt sich über eine entsprechende elastische Ausbildung des Deckelelements 86 im Bereich seiner Wände 100, 102 die jeweilige Rastnase 96, 98 in ihre zugeordnete Ausnehmung 106 bringen, wobei jedoch nach Einrasten in diese Ausnehmung 106 das Austauchen durch eine Wegschwenkung entgegen der Aufsetzrichtung gesperrt ist.

Um eine Vergrößerung des Querschnitts des ersten Teils 78 bei fixiertem Deckelelement 86 im Bereich der Rastverbindung 92 zu vermeiden, sind parallele Ausnehmungen 112 zur Aufnahme der Wände 100 und 102 vorgesehen, die dann entsprechend an die Dicke der Wände 100, 102 angepaßt sind, so daß bei fixiertem Deckelelement 86 Außenflächen der Wände 100, 102 im wesentlichen bündig in entsprechende weitere Außenflächen des ersten Teils 78, welche der Aufnahme 80 benachbart sind, übergehen.

Das erste Teil 78 ist in einem auf die Aufnahme 80 folgenden Kopplungsbereich 114 mit einem Zapfen 116 als Kopplungselement versehen. Dieser Kopplungsbereich 114 liegt dabei zwischen der Aufnahme 80 und einem Rastbereich 118, welcher mit dem Ausnehmungen 106 und 112 zur Aufnahme der Rastnasen 96, 98 und der Wände 100, 102 versehen ist.

Als zu dem Zapfen 116 korrespondierendes Kopplungselement ist in dem Deckelelement 86 eine Aufnahme 120 für den Zapfen 116 gebildet. Wenn das Deckelelement 86 über die Rastverbindung 92 an dem ersten Teil 78 drehfest fixiert ist, dann ist der Zapfen 116 in die Aufnahme 120 eingetaucht. Der Zapfen 116 erstreckt sich dabei in einer Richtung, welche quer und im wesentlichen senkrecht zu der Fläche 84 liegt. Bei dem gezeigten Ausführungsbeispiel ist der Zapfen 116 selber bereits in einem Abstand zu der Fläche 84 angeordnet, d. h. gegenüber dieser höhenversetzt.

Ist der Zapfen 116 in die Aufnahme 120 eingetaucht, dann ist quer zur Aufsetzrichtung des Deckelelements 86 auf das zweite Teil 79 die Bewegung des Deckelelements 86 zu dem ersten Teil 78 gesperrt. Die Rastverbindung 92 braucht dann nur erheblich kleinere Querkräfte aufzunehmen, so daß die Rastverbindung 92 im wesentlichen darauf hin optimierbar ist, daß eine Rastfixierung des Deckelelements 86 an dem ersten Teil 78 erreichbar ist, d. h. eine Wegschwenkung des Deckelelements 86 in Gegenrichtung zur Aufsetzrichtung des ersten Teils 78 auf das zweite Teil 79 gesperrt ist. Da die Querkräfte dann im wesentlichen durch die Kopplung des Zapfens 116 und die Zapfenaufnahme 120 aufnehmbar sind, müssen beispielsweise keine außergewöhnlichen Vorkehrungen getroffen werden, um für eine Querstabilität der Wände 100, 102 bezüglich des Verbindungsteils 104 zu sorgen. Dadurch wiederum lassen sich die Wände 100, 102 derart elastisch ausbilden und/oder elastisch an dem Verbindungsteil 104 anordnen, daß die Rastverbindung 92 durch Eintauchen der Rastnasen 96, 98 in die zugeordneten Ausnehmungen 112 auf einfache Weise erreichbar ist.

Ein entsprechendes medizinisches Instrument wird dann dadurch hergestellt, daß bei nicht fixiertem Deckelelement 86 (Figur 7), d. h. bei einem Deckelelement 86, welches so weit entgegen der Aufsetzrichtung weggeschwenkt ist, daß das zweite Teil 79 in die Aufnahme 80 und entsprechend beispielsweise mit einem Zapfen in die Zapfenaufnahme 82 einschiebbar ist, eben das zweite Teil 79 in die Aufnahme 80 gebracht wird. Es wird dann das Deckelelement 86 in der Aufsetzrichtung auf das zweite Teil 79 zu bewegt, d. h. beispielsweise auf dieses zu verschwenkt, und dabei die Rastverbindung 92 und die Kopplung des Zapfens 116 und der Aufnahme 120 hergestellt (Figur 8).

Gleichzeitig wird dadurch das Deckelelement 86 auf das zweite Teil 79 über die Fläche 90 aufgesetzt, um so die Bewegung des zweiten Teils 79 aus der Aufnahme 80 heraus in Richtung des Deckelelements 86 zu sperren.

## Patentansprüche

1. Medizinisches Instrument mit einem ersten Teil (12), einem zweiten Teil (14), welche über eine Verbindungsvorrichtung (16) miteinander verbunden sind, wobei das zweite Teil (14) an dem ersten Teil (12) anliegt, und mit einem Deckelelement (40), welches mit dem ersten Teil (12) verbunden ist und welches auf das zweite Teil (14) so aufgelegt ist, dass die Relativbewegung des zweiten Teils (14) von dem ersten Teil (12) weg über das Deckelelement (40) gesperrt ist, wobei ein Teil (12) mit einem Zapfen (18) versehen ist und das andere Teil (14) mit einer Zapfenausnehmung (28) zur Aufnahme des Zapfens (18) versehen ist, **dadurch gekennzeichnet , dass** der Zapfen (18) an dem entsprechenden Teil (12) einstückig angeformt ist, dass die Zapfenausnehmung (28) einen verbreiterten Bereich (34) aufweist, der einen größeren Querschnitt als der Zapfen (18) hat und in dem das Deckelelement (40) angeordnet ist, dass das Deckelelement (40) so ausgebildet ist, dass es nicht über eine Oberseite des zweiten Teils (14) hinausragt, und dass an der Zapfenausnehmung (28) durch den verbreiterten Bereich (34) eine Anlagefläche (38) für das Deckelelement (40) an das zweite Teil (14) angeordnet ist.

2. Medizinisches Instrument nach Anspruch 1, **dadurch gekennzeichnet, dass** ein Oberflächenbereich (24) des zweiten Teils (14) an einem zugeordneten Oberflächenbereich (20) des ersten Teils (12) anliegt.

3. Medizinisches Instrument nach Anspruch 1 oder 2, **dadurch gekennzeichnet, dass** ein Oberflächenbereich (42) des Deckelelements (40) an einem zugeordneten Oberflächenbereich (38) des zweiten Teils (14) anliegt.

4. Medizinisches Instrument nach einem der vorangehenden Ansprüche, **dadurch gekennzeichnet, dass** das Deckelelement (40) auf den Zapfen (18) aufgesetzt ist.

5. Medizinisches Instrument nach einem der vorangehenden Ansprüche, **dadurch gekennzeichnet, dass** das Deckelelement (40) in der Art eines Schnappverschlusses auf den Zapfen (18) aufsetzbar ist.

6. Medizinisches Instrument nach einem der vorangehenden Ansprüche, **dadurch gekennzeichnet, dass** der Zapfen (18) mit einem oder mehreren Längsschlitzen versehen ist, um eine Aufnahme für das Deckelelement (40) in der Art eines Schnappverschlusses zu bilden.

7. Medizinisches Instrument nach einem der vorangehenden Ansprüche, **dadurch gekennzeichnet, dass** das Deckelelement (40) mindestens im Bereich einer Anlagefläche (42) an das zweite Teil eine größere Querabmessung aufweist als der Zapfen (18).

8. Medizinisches Instrument nach einem der vorangehenden Ansprüche, **dadurch gekennzeichnet, dass** der Zapfen (18) an dem ersten Teil (12) angeordnet ist und die Zapfenausnehmung (28) an dem zweiten Teil (14) angeordnet ist.

9. Medizinisches Instrument nach einem der vorangehenden Ansprüche, **dadurch gekennzeichnet, dass** das zweite Teil (14) in einer Aufnahme (28) relativ zum ersten Teil (12) beweglich ist.

10. Medizinisches Instrument nach Anspruch 9, **gekennzeichnet durch** eine Führung (18, 28) des zweiten Teils (14) an dem ersten Teil (12).

11. Medizinisches Instrument nach einem der vorangehenden Ansprüche, **dadurch gekennzeichnet, dass** ein Teil (12) mindestens einen Zapfen (18) und das andere Teil (14) mindestens eine Zapfenaufnahme (28) zum Führen oder Halten des anderen Teils (14) an dem Teil (12) aufweist.

12. Medizinisches Instrument nach einem der vorangehenden Ansprüche, **dadurch gekennzeichnet, dass** die beiden Teile (12, 14) über die Verbindungsvorrichtung (16) relativ zueinander beweglich sind.

13. Medizinisches Instrument nach Anspruch 12, **dadurch gekennzeichnet, dass** die beiden Teile (12, 14) drehbar miteinander verbunden sind.

14. Medizinisches Instrument nach einem der vorangehenden Ansprüche, **dadurch gekennzeichnet, dass** das erste Teil (12) und/oder das zweite Teil (14) im wesentlichen aus einem Kunststoffmaterial hergestellt sind.

15. Medizinisches Instrument nach einem der vorangehenden Ansprüche, **dadurch gekennzeichnet, dass** das Deckelelement an dem ersten Teil fixierbar ist.

16. Medizinisches Instrument nach Anspruch 15, **dadurch gekennzeichnet, dass** das Deckelelement so an dem ersten Teil fixierbar ist, dass eine Wegbewegung von diesem gesperrt ist.

17. Medizinisches Instrument nach Anspruch 15 oder 16, **dadurch gekennzeichnet, dass** das Deckelelement an dem ersten Teil (78) über eine Rastverbindung fixierbar ist.

18. Medizinisches Instrument nach einem der vorangehenden Ansprüche, **dadurch gekennzeichnet, dass** das Deckelelement (40) zum Aufsetzen auf das zweite Teil (14) vor Fixierung mit dem ersten Teil (12) relativ zu dem ersten Teil (12) beweglich ist.

19. Medizinisches Instrument nach Anspruch 18, **dadurch gekennzeichnet, dass** das Deckelelement (40) ein separates Teil ist.

## Claims

1. Medical instrument with a first part (12), a second part (14) which are connected together by a connecting device (16), the second part (14) abutting on the first part (12), and with a cover element (40) which is connected to the first part (12) and is disposed on the second part (14) in such a manner that relative movement of the second part (14) away from the first part (12) is blocked by the cover element (40), one part (12) being provided with a spigot (18) and the other part (14) being provided with a spigot recess (28) for accommodating the spigot (18), **characterized in that** the spigot (18) is formed in one piece on the corresponding part (12), **in that** the spigot recess (28) has a widened portion (34) which is of greater cross section than the spigot (18) and in which the cover element (40) is disposed, **in that** the cover element (40) is formed in such a manner that it does not project above an upper face of the second part (14), and **in that** owing to the widened portion (34) a contact surface (38) for the cover element (40) on the second part (14) is arranged at the spigot recess (28).

2. Medical instrument in accordance with claim 1, **characterized in that** a portion (24) of the surface of the second part (14) abuts on an associated portion (20) of the surface of the first part (12).

3. Medical instrument in accordance with claim 1 or 2, **characterized in that** a portion (42) of the surface of the cover element (40) abuts on an associated portion (38) of the surface of the second part (14).

4. Medical instrument in accordance with any one of the preceding claims, **characterized in that** the cover element (40) is positioned on the spigot (18).

5. Medical instrument in accordance with any one of the preceding claims, **characterized in that** the cover element (40) can be placed on the spigot (18) in the manner of a snap-action closure.

6. Medical instrument in accordance with any one of the preceding claims, **characterized in that** the spigot (18) is provided with one or more longitudinal slots in order to form a seating for the cover element (40) in the manner of a snap-action closure.

7. Medical instrument in accordance with any one of the preceding claims, **characterized in that** the cover element (40) has a larger transverse dimension than the spigot (18) at least in the region of a contact surface (42) on the second part.

8. Medical instrument in accordance with any one of the preceding claims, **characterized in that** the spigot (18) is arranged on the first part (12) and the spigot recess (28) is arranged on the second part (14).

9. Medical instrument in accordance with any one of the preceding claims, **characterized in that** the second part (14) is moveable relative to the first part (12) in a seating (28).

10. Medical instrument in accordance with claim 9, **characterized by** a guiding (18, 28) for the second part (14) on the first part (12).

11. Medical instrument in accordance with any one of the preceding claims, **characterized in that** one part (12) comprises at least one spigot (18) and the other part (14) comprises at least one spigot seating (28) for guiding or holding the other part (14) on the one part (12).

12. Medical instrument in accordance with any one of the preceding claims, **characterized in that** the two parts (12, 14) are moveable relative to one another via the connecting device (16).

13. Medical instrument in accordance with claim 12, **characterized in that** the two parts (12, 14) are connected together in rotatable manner.

14. Medical instrument in accordance with any one of the preceding claims, **characterized in that** the first part (12) and/or the second part (14) are made substantially from a synthetic material.

15. Medical instrument in accordance with any one of the preceding claims, **characterized in that** the cover element is fixable to the first part.

16. Medical instrument in accordance with claim 15, **characterized in that** the cover element is fixable to the first part in such a manner as to block it from moving away therefrom.

17. Medical instrument in accordance with claim 15 or 16, **characterized in that** the cover element is fixable to the first part (78) by means of a latching connection.

18. Medical instrument in accordance with any one of the preceding claims, **characterized in that**, for the purpose of applying the cover element (40) to the second part (14) prior to being fixed to the first part (12), the cover element (40) is moveable relative to the first part (12).

19. Medical instrument in accordance with claim 18, **characterized in that** the cover element (40) is a separate part.

## Revendications

1. Instrument médical avec une première pièce (12) et une deuxième pièce (14) reliées l'une à l'autre par un mécanisme de liaison (16), la deuxième pièce (14) reposant contre la première pièce (12), et avec un élément de couverture (40) raccordé à la première pièce (12) et disposé sur la deuxième pièce (14) de telle manière que le déplacement relatif de la deuxième pièce (14) s'éloignant de la première pièce (12) soit bloqué par l'élément de couverture (40), une pièce (12) étant pourvue d'un tourillon (18) et l'autre pièce (14) d'un logement de tourillon (28) pour la réception du tourillon (18), **caractérisé en ce que** le tourillon (18) est formé d'une seul tenant sur la pièce (12) correspondante, **en ce que** le logement de tourillon (28) présente une zone évasée (34) de section transversale supérieure à celle du tourillon (18) et où est disposé l'élément de couverture (40), **en ce que** l'élément de couverture (40) est réalisé de manière à ne pas dépasser d'une face supérieure de la deuxième pièce (14), et **en ce qu'**une surface de butée (38) pour l'élément de couverture (40) est disposée sur la deuxième pièce (14), contre le logement de tourillon (28) en travers de la zone évasée (34).

2. Instrument médical selon la revendication 1, **caractérisé en ce qu'**une zone de surface (24) de la deuxième pièce (14) repose contre une zone de surface (20) correspondante de la première pièce (12).

3. Instrument médical selon la revendication 1 ou 2, **caractérisé en ce qu'**une zone de surface (42) de l'élément de couvertures (40) repose contre une zone de surface (38) correspondante de la deuxième pièce (14).

4. Instrument médical selon l'une des revendications précédentes, **caractérisé en ce que** l'élément de couverture (40) est monté sur le tourillon (18).

5. Instrument médical selon l'une des revendications précédentes, **caractérisé en ce que** l'élément de couverture (40) peut être monté sur le tourillon (18) à la manière d'une fermeture à enclenchement.

6. Instrument médical selon l'une des revendications précédentes, **caractérisé en ce que** le tourillon (18) est pourvu d'une ou de plusieurs fentes longitudinales permettant de former une réception pour l'élément de couverture (40) à la manière d'une fermeture à enclenchement.

7. Instrument médical selon l'une des revendications précédentes, **caractérisé en ce que** l'élément de couverture (40) présente une dimension transversale supérieure à celle du tourillon (18) au moins au niveau d'une surface de butée (42) sur la deuxième pièce.

8. Instrument médical selon l'une des revendications précédentes, **caractérisé en ce que** le tourillon (18) est disposé sur la première pièce (12) et **en ce que** le logement de tourillon (28) est disposé sur la deuxième pièce (14).

9. Instrument médical selon l'une des revendications précédentes, **caractérisé en ce que** la deuxième pièce (14) est mobile dans un logement (28) par rapport à la première pièce (12).

10. Instrument médical selon la revendication 9, **caractérisé par** un guidage (18, 28) de la deuxième pièce (14) contre la première pièce (12).

11. Instrument médical selon l'une des revendications précédentes, **caractérisé en ce qu'**une pièce (12) présente au moins un tourillon (18) et l'autre pièce (14) au moins un logement de tourillon (28) pour le guidage ou le maintien de l'autre pièce (14) contre la pièce (12).

12. Instrument médical selon l'une des revendications précédentes, **caractérisé en ce que** les deux pièces (12, 14) sont mobiles l'une par rapport à l'autre au moyen du mécanisme de liaison (16).

13. Instrument médical selon la revendication 12, **caractérisé en ce que** les deux pièces (12, 14) sont reliées l'une à l'autre de manière rotative.

14. Instrument médical selon l'une des revendications précédentes, **caractérisé en ce que** la première pièce (12) et/ou la deuxième pièce (14) sont essentiellement fabriquées dans le même matériau synthétique.

15. Instrument médical selon l'une des revendications précédentes, **caractérisé en ce que** l'élément de couverture peut être fixé sur la première pièce.

16. Instrument médical selon la revendication 15, **caractérisé en ce que** l'élément de couverture peut être fixé sur la première pièce de manière à bloquer un mouvement d'éloignement par rapport à celle-ci.

17. Instrument médical selon la revendication 15 ou 16, **caractérisé en ce que** l'élément de couverture peut être fixé sur la première pièce (78) par une connexion à enclenchement.

18. Instrument médical selon l'une des revendications précédentes, **caractérisé en ce que** l'élément de couverture (40) est mobile par rapport à la première pièce (12) pour le montage sur la deuxième pièce (14) avant la fixation avec la première pièce (12).

19. Instrument médical selon la revendication 18, **caractérisé en ce que** l'élément de couverture (40) est une pièce séparée.
